(19) Europäisches Patentamt
European Patent Office
Office européen des brevets

(11) **EP 4 309 579 A1**

(12) **EUROPEAN PATENT APPLICATION**
published in accordance with Art. 153(4) EPC

(43) Date of publication:
**24.01.2024 Bulletin 2024/04**

(21) Application number: **22770154.7**

(22) Date of filing: **15.03.2022**

(51) International Patent Classification (IPC):
**A61B 5/11** (2006.01)    **A61B 5/00** (2006.01)
**A63B 24/00** (2006.01)

(52) Cooperative Patent Classification (CPC):
**A61B 5/00; A61B 5/103; A61B 5/11; A63B 24/00**

(86) International application number:
**PCT/CL2022/050022**

(87) International publication number:
**WO 2022/193034 (22.09.2022 Gazette 2022/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.03.2021 CL 20210634**

(71) Applicant: **Universidad de Talca
Talca (CL)**

(72) Inventors:
• **SOTO POBLETE, Alex Eduardo
Villa Santa María de Colín Talca (CL)**

• **IBARRA CORTÉS, Natalia Audoriza
501 Talca (CL)**
• **MARTÍNEZ DÍAZ, Darián Róbinson
Hualañé (CL)**
• **FERNÁNDEZ FERNÁNDEZ, Mario Alberto
Condominio Altos del Boldo Curicó (CL)**
• **ASTUDILLO HERNÁNDEZ, César Alejandro
Villa Santa Ana del Boldo Curicó (CL)**
• **GATICA ROJAS, Valeska Fabiola
Talca (CL)**
• **GONZÁLEZ NÚÑEZ, Marco Antonio
Talca (CL)**

(74) Representative: **Ungria López, Javier
Avda. Ramón y Cajal, 78
28043 Madrid (ES)**

(54) **SYSTEM FOR DETECTING POSTURAL PROBLEMS AND LOSS OF BALANCE**

(57)    The present invention provides a portable, easy-to-use system for detecting postural problems and loss of balance in a user comprising a t-shirt including an inertial sensor; a head-mounted frame comprising a micro camera and an inertial microsensor, and an electronic device connected to the micro camera, the inertial microsensor of the frame, and the inertial sensor of the t-shirt, wherein the electronic device is configured to receive information of the user's head and eyes from the micro camera and the inertial microsensor of the frame, to receive information of the movement and force performed by said user through the inertial sensor of the t-shirt, and to process said information to obtain the user's center of pressure in real time. This system allows early detection, prevention, and diagnosis of loss of balance, fall risks, and alterations in postural control.

FIG. 1

## Description

## Technical field of the invention

The present invention relates to the technical field of detecting, measuring, or recording devices for testing the shape, pattern, color, size or movement of the body or parts thereof for diagnostic purposes, in particular to sensing devices and methods that can be used to estimate the risk of falling. Specifically, it provides a system for detecting postural problems and loss of balance.

## Invention background

The world's population is aging at a rapid pace; between 2000 and 2050 the proportion of the world's population over the age of 60 will double from 11% to 22%. In older adults, balance is continually compromised, decreasing their functional independence and quality of life, and is a risk factor for falls and hip fractures, among other traumatic events. Additionally, older people who are prone to falls are more likely to have abnormalities in postural stability and balance control.

Among the solutions described in the prior art are, for example, US 2014/024972, which describes a system for assessing fall risks comprising: an inertial sensor able to be attached to a person; a pressure sensor matrix having a plurality of pressure sensors, each of the plurality of pressure sensors configured to sense a pressure exerted by the person; one or more processors configured to receive inertial sensor data from the inertial sensor and to receive pressure sensor data from the pressure sensor matrix, wherein the one or more processors are configured to: generate center of pressure data based on the received pressure sensor data; receive a value indicative of whether the person has fallen during a predetermined interval, generate a classifier function based on the inertial sensor data, the pressure sensor data, and the value, and generate with said classification function, indicative fall risk values based on other pressure sensor data and other inertial sensor data.

On the other hand, WO 2013/165557 provides an exercise gaming system, comprising a motion-detect device including a plurality of sensors configured to monitor movement of the motion-detect device during a user motion, a first wireless communication interface, a first processor configured to receive input data from the plurality of sensors and provide information related to the data for transmission via the wireless communication interface, and a computing device including a second wireless communication interface configured to receive information transmitted from the motion-detect device, a first memory, a second processor configured to compare the received information to data stored in the memory and identify the user motion as a predefined movement, and a display configured to visually provide feedback related to the identified predefined movement.

However, the solutions described in the prior art do not disclose a system for detecting postural problems and loss of balance, adaptable to everyday garments or accessories, so as to monitor posture and loss of balance at all times, without the need of adding additional devices, but that are already incorporated in said everyday garments or accessories.

Consequently, a portable and easy-to-use technology is required, such as an intuitive, lightweight, ergonomic, and intelligent sports garment, at an accessible cost, and that allows early detection, prevention, and diagnosis of loss of balance, fall risks and postural control alterations.

## Invention summary

The present invention provides a system for detecting postural problems and loss of balance characterized in that it comprises a t-shirt comprising an inertial sensor; a head-mounted frame comprising a micro camera and an inertial microsensor positioned frontally on said frame, and a first controller operatively connected to said micro camera and to said inertial microsensor; an electronic device wirelessly connected to said micro camera and to said inertial microsensor of said frame, and connected to said inertial sensor of said t-shirt, wherein said electronic device comprises a first processor, a first communication interface operatively connected to said first processor; a second controller comprising a second processor, a storage memory operatively connected to said second processor, a second communication interface operatively connected to said second processor and in communication with said first communication interface, and a user interaction interface operatively connected to said second processor; wherein said second controller is configured to receive information of the head and eyes of a user from said micro camera and from said inertial microsensor of said frame, and to receive information of the movement and force performed by said user through said inertial sensor of said t-shirt, and to process said information to obtain the user's center of pressure in real time.

In a preferred embodiment, the system for detecting postural problems and loss of balance is characterized in that said t-shirt comprises a plurality of inertial sensors, and in that said electronic device is operatively connected to each of the inertial sensors forming said plurality of inertial sensors.

In another preferred embodiment, the system for detecting postural problems and loss of balance is characterized in that said t-shirt comprises five inertial sensors.

In another preferred embodiment, the system for detecting postural problems and loss of balance is characterized in that a first inertial sensor is positioned in the sternal manubrium area, a second inertial sensor is positioned in the left acromion area, a third inertial sensor is positioned in the right acromion area, a fourth inertial sensor is positioned in the C7 spinous process area, and a fifth inertial sensor is positioned in the L4 spinous process area.

In a further preferred embodiment, the system for detecting postural problems and loss of balance is characterized in that said electronic device is connected to said inertial sensor through thermoset insulated cables.

**Brief description of figures**

FIG. 1 shows a front view of a first embodiment of the sensorized t-shirt that is part of the system for detecting postural problems and loss of balance that is the subject of the present invention.

FIG. 2 shows a rear view of a first embodiment of the sensorized t-shirt that is part of the system for detecting postural problems and loss of balance that is the subject of the present invention.

FIG. 3 shows a main view of a first embodiment of a head-mounted frame that is part of the system for detecting postural problems and loss of balance that is the subject of the present invention.

FIG. 4 shows a main view of two embodiments of a second controller that is part of the system for detecting postural problems and loss of balance that is the subject of the present invention.

**Detailed description of the invention**

Essentially, the present invention provides a system for detecting postural problems and loss of balance comprising: a t-shirt (1) comprising an inertial sensor (11); a head-mounted frame (2) comprising a micro camera (21) and an inertial microsensor (22) positioned frontally on said frame (2), and a first controller operatively connected to said micro camera (21) and to said inertial microsensor (22); an electronic device (3) wirelessly connected to said micro camera (21) and to said inertial microsensor (22) of said frame (2), and connected to said inertial sensor (11) of said t-shirt (1), wherein said electronic device (3) comprises a first processor, a first communication interface operatively connected to said first processor; a second controller (4) comprising a second processor, a storage memory operatively connected to said second processor, a second communication interface operatively connected to said second processor and in communication with said first communication interface, and a user interaction interface operatively connected to said second processor; wherein said second controller (4) is configured to receive information of the head and eyes of a user from said micro camera (21) and from said inertial microsensor (22) of said frame (2), and to receive movement and force information performed by said user through said inertial sensor (11) of said t-shirt (1), and to process said information to obtain the user's center of pressure in real time.

In the context of the present invention, and without limiting the scope of the protection, it will be understood as frontally positioned a position located at the front of the head-mounted frame (2), however, with the particularity that its orientation will be in the opposite direction to the user's vision; given that the data of interest are obtained from the user's gaze and head movements. In other words, it can be positioned in any frontal and rear area of the head-mounted frame (2), so as to correctly measure data from the eyes and head of a user.

The size of said t-shirt (1) does not limit the scope of the present invention. They may be, for example and without being limited thereto, standard sizes, e.g., S, M, L, XL. Likewise, the material of said t-shirt (1) does not limit the scope of the present invention. It may be, for example and without being limited thereto, cotton, polyester, silk, linen, or any of their derivatives and even some mixture thereof. In an advantageous embodiment, without limiting the scope of the present invention, said t-shirt (1) is made of 100% polyester fabric. In a preferred embodiment, without limiting the scope of the present invention, the t-shirt (1) may comprise a plurality of inertial sensors (11a, 11b, 11c), wherein each of the sensors forming said plurality of inertial sensors (11a, 11b, 11c) are operatively connected to said electronic device (3). The number of sensors comprising said t-shirt (1) is not limiting feature. In a preferred embodiment, and without limiting the scope of protection, said t-shirt (1) may comprise between two and ten inertial sensors. In a more preferred embodiment, said t-shirt (1) comprises five inertial sensors (11a, 11b, 11c, 11d, 11e).

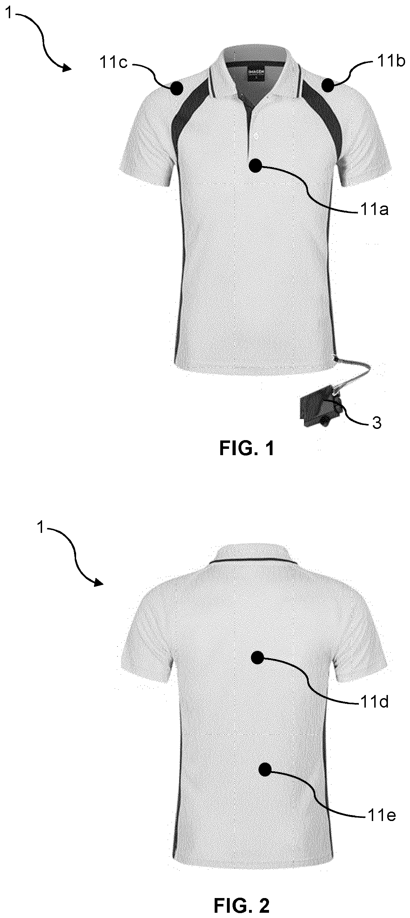

On the other hand, the position within said t-shirt (1) in which said inertial sensor (11) is disposed does not limit the scope of the present invention. In those embodiments in which the t-shirt (1) comprises a plurality of inertial sensors (11a, 11b, 11c, 11d, 11e), the manner in which each of the sensors forming said plurality of inertial sensors (11a, 11b, 11c, 11d, 11e) are distributed does not limit the scope of the present invention. In a preferred embodiment, and without limiting the scope of protection, three inertial sensors (11a, 11b, 11c) are positioned on the frontal area of said t-shirt (1) and two sensors (11d, 11e) are positioned on the rear area of t-said shirt (1). In an example embodiment, without limiting the scope of the present invention, a first inertial sensor (11a) is positioned in the sternal manubrium area, a second inertial sensor (11b) is positioned in the left acromion area, a third inertial sensor (11c) is positioned in the right acromion area, a fourth inertial sensor (11d) is positioned in the C7 spinous process area, and a fifth inertial sensor (11e) is positioned in the L4 spinous process area; as shown in FIGs. 1 and 2. Said distribution is advantageous in that it captures the different vectors in the planes of motion and positions of the body in space. Thus, without limiting the scope of the present invention, it is possible to build the postural control of a user when standing still (static) or during movement (dynamic) from said distribution.

Additionally, the type, size, and brand of each of the inertial sensors forming said plurality of inertial sensors (11a, 11b, 11c, 11d, 11e) do not limit the scope of the present invention. They may be, for example and without being limited thereto, a gyroscope, an accelerometer, a magnetometer, among others; or an arrangement of two or more thereof. In a preferred embodiment, and without limiting the scope of protection, each of the inertial sensors forming said plurality of inertial sensors (11a, 11b, 11c, 11d, 11e) are an arrangement of a 3-axis accelerometer gyroscope plus a 3-axis magnetometer. In a more preferred embodiment, each of the inertial sensors forming said plurality of inertial sensors (11a, 11b, 11c, 11d, 11e) are an arrangement resulting from the combination of an *MPU-6500 sensor for Arduino* plus an *AK8963 magnetometer for Arduino.*

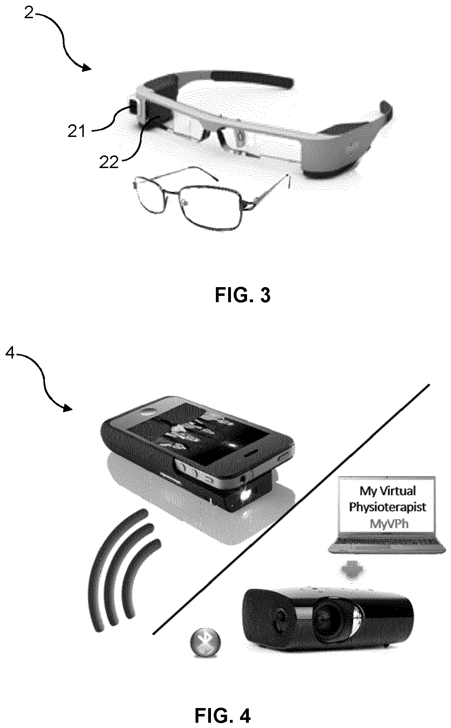

The head-mounted frame (2) corresponds to a housing adaptable to a typical accessory for human use. The shape, material, and size of said head-mounted frame (2) do not limit the scope of the present invention. Said frame (2) may, for example and without being limited thereto, be selected from the group consisting of eyeglass frames, goggles, face shields, caps, hats, helmets, masks, among others, as well as a combination thereof. In a preferred embodiment, and without limiting the scope of protection, said head-mounted frame (2) is a plastic eyeglass frame, as shown in FIG. 3. On the other hand, said head-mounted frame (2) comprises a micro camera (21) and an inertial microsensor (22) positioned frontally. The type, size, and brand of both said micro camera (21) and said inertial microsensor (22) do not limit the scope of the present invention. In a preferred embodiment, and without limiting the scope of protection, said micro camera (21) is a *CMOS camera module for Arduino* and said inertial microsensor (22) is an infrared reflectance sensor module for *Arduino.* Additionally, said micro camera (21) and said inertial microsensor (22) of said head-mounted frame (2) are operatively connected to a first controller. The type, size, and brand of said first controller, does not limit the scope of the present invention. It may be, for example and without being limited thereto, an *ATmega328,* an *AVR mini,* an *Arduino,* among others. In a preferred embodiment, and without limiting the scope of protection, said first controller is an *Arduino Nano* board.

The electronic device (3) comprises, essentially, a processor and energy storage means; generally, lithium polymer batteries. The type, size, and brand of the processor contained in said electronic device (3) does not limit the scope of the present invention. In a preferred embodiment, without limiting the scope of the present invention, said processor may be chosen from the group formed by an *ATmega328,* an *AVR mini,* an *Arduino,* among others. In a more preferred embodiment, and without limiting the scope of protection, said processor is an *Arduino Nano* board.

On the other hand, said electronic device (3) is connected to the inertial sensor (11) of the t-shirt (1). In the context of the present invention, without limiting the scope thereof, it will be understood that said electronic device (3) is connected to an inertial sensor (11) when the processor of said electronic device (3) can obtain measurements from said inertial sensor (11). Said connection may be wired or wireless, without limiting the scope of the present invention. In those preferred embodiments in which the t-shirt (1) comprises a plurality of inertial sensors (11a, 11b, 11c, 11d, 11e), said electronic device (3) is connected to each of the inertial sensors forming said plurality of inertial sensors (11a, 11b, 11c, 11d, 11e). In a preferred embodiment, and without limiting the scope of protection, said electronic device (3) is connected to each of the inertial sensors forming said plurality of inertial sensors (11a, 11b, 11c, 11d, 11e) in a wired manner. In a more preferred embodiment, said electronic device (3) is connected to each of the inertial sensors forming said plurality of inertial sensors (11a, 11b, 11c, 11d, 11e) through thermoset insulated wires.

The second controller (4) essentially comprises a second processor, a storage memory operatively connected to said second processor, a second communication interface operatively connected to said second processor and in communication with said first communication interface, and a user interaction interface operatively connected to said second processor.

The type, size, and brand of said second controller (4) does not limit the scope of the present invention. In a preferred embodiment, and without limiting the scope of protection, said second controller (4) may be a smart device, such as, for example and without limiting the scope of the present invention, a smartphone, a smartwatch, or a portable tablet. In a more preferred embodiment, without limiting the scope of the present invention, said second controller (4) is a smartphone with an application and a projection device. In an alternative preferred embodiment, and without limiting the scope of protection, said second controller (4) is a computer with a program and a projector; as shown in FIG. 4.

Additionally, said second controller (4) is configured to receive information from said user's head movements through said inertial microsensor (22) of said frame (2) and to receive information from said user's eyes through said micro camera (21) of said frame (2); emulating an eye tracking device, in addition to quantifying said user's eye temperature, gaze focus and decision making process. As well as to receive information of movement and body force of said user through each of the inertial sensors forming said plurality of inertial sensors (11a, 11b, 11c, 11d, 11e) positioned on the t-shirt (1), and to process said information to obtain the center of pressure of said user at all times.

The algorithm or formulas used for programming the second controller (4) do not limit the scope of the present invention. In a preferred embodiment, and without limiting the scope of protection, said controller uses the following formulas to obtain the coordinates of the center of pressure:

$$Cp_{ap} = \frac{(-h * Fx - My)}{Fz}$$

$$Cp_{ml} = \frac{(-h * Fy - Mx)}{Fz}$$

Wherein the terminology "ap" and "ml" mean anterior-posterior direction and medio-lateral direction, respectively. And each of the inertial sensors forming said plurality of inertial sensors (11a, 11b, 11c, 11d, 11e) measure three force components Fx, Fy, Fz, and three moment components Mx, My, Mz.

Additionally, the eye-tracking algorithms used do not limit the scope of the present invention. For example, and without limiting the scope of the present invention, said second controller (4) may be configured to determine, from the information provided by said micro camera (21), whether the gaze is fixed or producing saccadic movements. In another example embodiment, without limiting the scope of the present invention, said second controller (4) may execute an algorithm that is selected from the group formed by Infrared Oculography (IOG), Video Oculography (VOG) techniques, as well as a combination thereof.

Additionally, from the center of pressure and eye position information, said second processor (4) may be configured, without limiting the scope of the present invention, to obtain a postural construct containing the eye, head, and trunk movements, and thus the overall/total posture (postural control) of the user.

In this way, it is possible to obtain a system for the detection of postural problems and loss of balance that allows to solve the problems previously exposed.

**Claims**

1. A system for detecting postural problems and loss of balance, **CHARACTERIZED in that** it comprises:

   - a t-shirt (1) comprising an inertial sensor (11);
   - a head-mounted frame (2) comprising a micro camera (21) and an inertial microsensor (22) positioned frontally on said frame (2), and a first controller operatively connected to said micro camera (21) and to said inertial microsensor (22);
   - an electronic device (3) wirelessly connected to said micro camera (21) and to said inertial microsensor (22) of said frame (2), and connected to said inertial sensor (11) of said t-shirt (1);

   wherein said electronic device (3) comprises a first processor, a first communication interface operatively connected to said first processor;
   - a second controller (4) comprising a second processor, a storage memory operatively connected to said second processor, a second communication interface operatively connected to said second processor and in communication with said first communication interface, and a user interaction interface operatively connected to said second processor; wherein said second controller (4) is configured to receive information of the head and eyes of a user from said micro camera (21) and from said inertial microsensor (22) of said frame (2), and to receive movement and force information performed by said user through said inertial sensor (11) of said t-shirt (1), and to process said information to obtain the user's center of pressure in real time.

2. The system for detecting postural problems and loss of balance according to claim 1, **CHARACTERIZED in that** said t-shirt (1) comprises a plurality of inertial sensors (11a, 11b, 11c); and **in that** said electronic device (3) is operatively connected to each of the inertial sensors forming said plurality of inertial sensors (11a, 11b, 11c).

3. The system for detecting postural problems and loss of balance according to claim 2, **CHARACTERIZED in that** said t-shirt (1) comprises five inertial sensors (11a, 11b, 11c, 11d, 11e).

4. The system for detecting postural problems and loss of balance according to claim 3, **CHARACTERIZED in that** a first inertial sensor (11a) is positioned in the sternal manubrium area, a second inertial sensor (11b) is positioned in the left acromion area, a third inertial sensor (11c) is positioned in the right acromion area, a fourth inertial sensor (11d) is positioned in the C7 spinous process area, and a fifth inertial sensor (11e) is positioned in the L4 spinous process area.

5. The system for detecting postural problems and loss of balance according to claim 1, **CHARACTERIZED in that** said electronic device (3) is connected to said inertial sensor (11) through thermoset insulated wires.

1

11c — 11b

11a

3

**FIG. 1**

1

11d

11e

**FIG. 2**

**FIG. 3**

**FIG. 4**

## INTERNATIONAL SEARCH REPORT

| International application No. |
|---|
| PCT/CL2022/050022 |

### A. CLASSIFICATION OF SUBJECT MATTER

IPC(8) - A61B 5/11; A61B 5/00; A63B 24/00 (2022.01)

CPC - A61B 5/1126; A61B 5/11; A61B 5/1116; A61B 5/68; A61B 5/6801; A63B 24/00 (2022.05)

According to International Patent Classification (IPC) or to both national classification and IPC

### B. FIELDS SEARCHED

Minimum documentation searched (classification system followed by classification symbols)

see Search History document

Documentation searched other than minimum documentation to the extent that such documents are included in the fields searched

see Search History document

Electronic data base consulted during the international search (name of data base and, where practicable, search terms used)

see Search History document

### C. DOCUMENTS CONSIDERED TO BE RELEVANT

| Category* | Citation of document, with indication, where appropriate, of the relevant passages | Relevant to claim No. |
|---|---|---|
| Y | US 2009/0062092 A1 (MORTIMER et al) 05 March 2009 (05.03.2009) entire document | 1-5 |
| Y | US 2013/0307842 A1 (GRINBERG et al) 21 November 2013 (21.11.2013) entire document | 1-5 |
| Y | PATIAS et al. A review of the trunk surface metrics used as Scoliosis and other deformities evaluation indices. Scoliosis 2010, 5(12) . [retrieved on 31.05.2022]. Retrieved from the Internet. <URL: https://scoliosisjournal.biomedcentral.com/articles/10.1186/1748-7161-5-12>. entire document | 4 |
| Y | WO 2017/151523 A1 (LIQUID WIRE LLC et al) 08 September 2017 (08.09.2017) entire document | 5 |

☐ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 31 May 2022 | JUN 16 2022 |

| Name and mailing address of the ISA/US | Authorized officer |
|---|---|
| Mail Stop PCT, Attn: ISA/US, Commissioner for Patents P.O. Box 1450, Alexandria, VA 22313-1450 | Taina Matos |
| Facsimile No. 571-273-8300 | Telephone No. PCT Helpdesk: 571-272-4300 |

Form PCT/ISA/210 (second sheet) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014024972 A

- WO 2013165557 A